# EUROPÄISCHE PATENTANMELDUNG

(11) **EP 3 153 494 A1**
(43) Veröffentlichungstag der Anmeldung: **12.04.2017**
(21) Anmeldenummer: 15188870.8
(22) Anmeldetag: 08.10.2015
(51) Int. Cl.: C07C 45/32, C07C 49/587

(54) **VERWENDUNG NEUARTIGER GEMISCHE VON (E/Z)-CYCLOPENTADECENON-ISOMEREN, DEREN HERSTELLUNG UND DEREN VERWENDUNG ALS AROMASTOFF**

(71) Anmelder: BASF SE, 67056 Ludwigshafen am Rhein (DE)
(72) Erfinder: THRUN, Frauke ,, 68163 Mannheim (DE); TELES, Joaquim Henrique,, 67165 Waldsee (DE); WERNER , Albert,, 67245 Lambsheim (DE); PELZER, Ralf,, 37699 Fuerstenberg (DE)
(74) Vertreter: Reitstötter Kinzebach

(57) **Zusammenfassung**

Die vorliegende Erfindung betrifft neuartige Gemische von Cyclopentadecenon-Isomeren, deren Herstellung und deren Verwendung als Aromastoff, insbesondere als Riechstoff, sowie Aromastoffzusammensetzungen und Mittel, enthaltend diese Gemische.

## Beschreibung

Die vorliegende Erfindung betrifft neuartige Gemische von (E/Z)-Cyclopentadecenon-Isomeren, deren Herstellung und deren Verwendung als Aromastoff, insbesondere als Riechstoff, sowie Aromastoffzusammensetzungen und Mittel, enthaltend diese Gemische.

### Hintergrund der Erfindung

In der Parfümindustrie besteht ein ständiger Bedarf an neuen Riechstoffen, die sich als Riechstoffkompositionen oder parfümierten Artikeln eignen.

Insbesondere besteht ein Bedarf an moschusartigen Riechstoffen und Riechstoffkompositionen. Hierunter ist ein Geruch zu verstehen, der dem natürlich vorkommenden Moschusduft ähnlich ist.

*Trans-* und *cis*-Cyclopentadec-8-enon finden bereits Erwähnung in US 5,936,100 und in Fürstner, A. et al, Synthesis 1997, 792. Die Verbindungen wurden ausgehend von Heptadeca-1,16-dienon mittels einer Ruthenium-katalysierten Ringschlussalkenmetathese hergestellt. In der EP-A-1 264 594 wird die Verwendung von *cis-* und *trans*-Cyclopentedec-7-enon als Beimischung in einer Formulierung zur Inhibierung der Melaninsynthese erwähnt. Die EP-A 216 185 erwähnt, dass die Verbindungen der *cis*-7- und -8-Cyclopentadecen-1-one ausgehend von *cis*-16-Oxabicyclo-[13.1.0]-hexadec-8-en über eine Säure oder Lewis-Säure katalysierte Meerwein-Umlagerung hergestellt werden können, ohne hierzu jedoch eine experimentellen Beleg zu liefern. Ferner wird die Brauchbarkeit der beiden Verbindungen als Duftstoff postuliert. Die tatsächlichen olfaktorischen Eigenschaften der 7- bzw. 8-Cyclopentadecenone wurden jedoch in der Literatur bisher noch nicht beschrieben.

Die Cu katalysierte oxidative Decarbonylierung ist bekannt und vielfältig publiziert, Beispiele finden sich: a) Tetrahedron Letters 1969, 12, 985; US3496197; b) Tetrahedron Letters 1995, 4641, c) Org. Lett. 2010, 2630, d) Bioorg. Med. Chem. Lett. 2013, 23, 5949, e) Chin. Chem. Lett. 2014, 25, 771.

Aus dem Stand der Technik ist weiterhin die Oxidation von Cyclohexadeca-1,9-dien mit N₂O bekannt (WO 2012/084673). Dabei entstehen als Hauptprodukt (E/Z)-Cyclohexadec-8-enon sowie als Nebenprodukte verschiedene Cyclopentadecenylcarbaldehyde. Eine destillative Trennung des Gemisches aus Cyclohexadec-8-enon und Cyclopentadecenylcarbaldehyd ist nur mit einem hohen Ausbeuteverlust an Cyclohexadec-8-enon möglich. Eine Trennung des Gemisches aus Cyclohexadec-8-enon und Cyclopentadecenylcarbaldehyd muss aber erfolgen, da sonst Cyclohexadec-8-enon mit einer Fehlnote behaftet ist.

Aufgabe der vorliegenden Erfindung ist die Bereitstellung neuartiger moschusartiger Duftstoffe.

### Zusammenfassung der Erfindung

Überraschenderweise wurde obige Aufgabe insbesondere durch Bereitstellung der in den Ansprüchen definierten makrocyclischen Ketoverbindungen und eines Verfahrens zu deren Herstellung und Isolierung gelöst.

Es wurde insbesondere gefunden, dass die cyclischen Aldehyde, welche als Nebenprodukte in der Oxidation von Cyclohexadeca-1,9-dien mit Lachgas gebildet werden, einer Cu katalysierten, oxidativen Decarbonylierung unterworfen werden können. Aus dem so erhaltenen Reaktionsaustrag lässt sich Cyclohexadec-8-enon destillativ von den entstandenen Cyclopentadec-8-enonen ohne wesentliche Ausbeuteverluste trennen, zusätzlich lässt sich bei der Destillation ein Gemisch im Wesentlichen bestehend aus (E/Z)-Cyclopentadec-8-enon (Verhältnis z.B. 1:1) und (E/Z)-Cyclopentadec-7-enon (Verhältnis 1:1), wobei das Verhältnis von Cyclopentadec-8-enon und Cyclopentadec-7-enon 10 : 1 beträgt, mit wertvollen olfaktorischen Eigenschaften isolieren.

Es wurde überraschenderweise gefunden, dass die Cyclopentadecenone **I-IV** aus der Cu katalysierten, oxidativen Decarbonylierung von Cyclopentadecenylcarbaldehyd **XI** olfaktorisch sehr gut riechen. Die Cyclopentadecenone **I-IV** erweitern somit das Repertoire der Duftstoffindustrie, insbesondere der moschusartigen Riechstoffe. Die Verbindungen zeichnen sich durch einen grün, aldehydisch, harsch, gasig, süß, pudrig, nach Trockenobst und nach Moschus riechenden Geruch aus. Die Cyclopentadec-8-enone fallen als (E/Z)-Mischung im Verhältnis 1:1 in der Cu katalysierten, oxidativen Decarbonylierung an. Als Nebenprodukte wurden auch die (E/Z)-Cyclopentadec-7-enon erhalten.

### Detaillierte Beschreibung der Erfindung

### a) Allgemeine Definitionen:

Eine "Aromachemikalie" ist ein Oberbegriff für Verbindungen die als "Riechstoff" und/oder als "Geschmackstoff" einsetzbar sind.

Als "Riechstoff" sind im Sinne der vorliegenden Erfindung natürliche oder synthetische Stoffe mit Eigengeruch zu verstehen.

Als "Geschmackstoff" sind im Sinne der vorliegenden Erfindung natürliche oder synthetische Stoffe mit Eigengeschmack zu verstehen.

Der "Geruch" oder die "olfaktorische Wahrnehmung" ist im Sinne der vorliegenden Erfindung die Interpretation der Sinneserregungen, die von den Chemorezeptoren der Nase oder anderen Geruchsorganen an das Gehirn eines Lebewesens geliefert werden. Der Geruch kann folglich eine Sinneswahrnehmung der Nase von Riechstoffen sein, die beim Einatmen erfolgt. Die Luft dient hierbei als Geruchsträger.

Als "Duft" ist im Sinne der vorliegenden Erfindung ein wohlriechender Geruch zu verstehen. Entsprechendes gilt für einen erfindungsgemäßen "Duftstoff".

Ein "Parfüm" ist im Sinne der vorliegenden Erfindung ein Gemisch aus Riechstoffen und Träger, wie insbesondere einem Alkohol.

Eine "Parfümzusammensetzung" ist im Sinne der vorliegenden Erfindung ein Parfüm, welches unterschiedliche Mengen harmonisch aufeinander abgestimmter Einzelkomponenten enthält. Die Eigenschaften der Einzelbestandteile werden genutzt, um in der Kombination ein neues Gesamtbild zu schaffen, wobei die Charakteristika der Ingredienzen in den Hintergrund treten, ohne jedoch unterdrückt zu werden.

Ein "Parfümöl" ist im Sinne der vorliegenden Erfindung ein konzentriertes Gemisch aus mehreren Riechstoffen, die z.B. in alkoholischen Lösungen zur Parfümierung verschiedener Produkte gebraucht werden.

Ein "Duftthema" ist im Sinne der vorliegenden Erfindung die vorherrschende Duftnote in einer Riechstoffzusammensetzung.

Die "Kopfnote" ist im Sinne der vorliegenden Erfindung die erste Phase des Duftablaufs eines Parfüms. Sie spielt die ausschlaggebende Rolle beim ersten Eindruck, beim Öffnen des Flakons und beim Auftragen des Parfüms auf die Haut. Die Aufgabe der Kopfnote ist es, Interesse für das Parfüm allgemein zu wecken und für Aufmerksamkeit zu sorgen. Deshalb ist ein außergewöhnlicher Charakter häufig wichtiger als eine ausgefeilte Harmonie. Die Kopfnote wird naturgemäß von leichtflüchtigen Riechstoffen bestimmt.

"Modifizieren" bedeutet im Sinne der vorliegenden Erfindung, das Grundthema einer Riechstoffzusammensetzung mit zusätzlichen oder anderen Akkorden und Geruchsnuancen zu versehen.

"Akkorde" entstehen im Sinne der vorliegenden Erfindung durch das Zusammenfügen verschiedener Riechstoffe, die sich somit zu neuen Geruchsbildern vereinigen. Die Anzahl der eingesetzten Riechstoffe kann von zwei bis zu hundert Verschiedene reichen.

Eine "organoleptisch/sensorisch wirksame Menge" im Sinne der vorliegenden Erfindung ist die Menge eines Riechstoffes, die ausreicht, um anregend auf ein Sinnesorgan beziehungsweise anregend auf einen sensorischen Rezeptor zu wirken.

### b) Spezielle Ausführungsformen der Erfindung

Vorliegende Erfindung betrifft insbesondere folgende Gegenstände:
1. Verfahren zur Herstellung wenigstens einer makrocyclischen, insbesondere einkernigen, makrocyclischen Ketoverbindung der allgemeinen Formel **X** 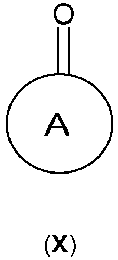 worin A für einen cycloaliphatischen Kohlenwasserstoffrest mit m Ringkohlenstoffatomen steht, wobei m für einen ganzzahligen Wert von 13 bis 17, wie z.B. 13, 14, 15, 16 oder 17, insbesondere 15, steht, und gegebenenfalls n C=C-Doppelbindungen aufweist, wobei n für einen ganzzahligen Wert von 1, 2 oder 3, insbesondere 1 steht
   wobei man
   a) Wenigstens eine cycloaliphatische Carbaldehydverbindung der Formel **XI** 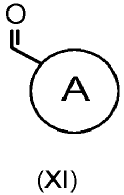 worin A die oben angegebenen Bedeutungen besitzt, oxidativ decarbonyliert; wobei man die Verbindung der Formel **XI** in Gegenwart eines, vorzugsweise einer katalytischen Menge eines vorzugsweise homogenen Cu-(II)-Katalysators und molekularem reinem Sauerstoff, Magerluft oder vorzugsweise Luft, insbesondere getrockneter, filtrierter Umgebungsluft, umsetzt; und gegebenenfalls
   b) wenigstens eine Verbindung der Formel **X** aus dem Reaktionsgemisch isoliert;
   Dabei erhält man die Zielverbindung der Formel **X** in stereoisomerenreiner Form oder in Form von Stereoisomerengemischen, enthaltend wenigstens 2, insbesondere 2, 3 oder 4, stereoisomere Formen solchen Ketoverbindungen; oder wobei man Stoffgemische erhält, enthaltend wenigstens 2 wie z.B. 2, 3, oder 4, insbesondere 2 oder 3, derartige Ketoverbindungen in jeweils stereoisomerenreiner Form oder als Stereoisomerengemisch. Insbesondere erhält man bevorzugt Verbindungen, worin im Falle von n=1 die Ketogruppe und die Ring-C=C-Doppelbindung 4 bis 7 Ring-C-Atome voneinander entfernt sind. Stoffgemische können, wenn n nicht für 0 steht auch Konstitutionsisomere umfassen. Falls n für 2 oder 3 steht dann sind die Doppelbindungen nicht kumuliert.
2. Verfahren nach Ausführungsform 1, wobei die oxidative Decarbonylierung zusätzlich in Gegenwart einer insbesondere organischen Base erfolgt.
   Die im erfindungsgemäßen Verfahren beispielsweise eingesetzte Base ist beispielsweise ausgewählt unter Diazabicycloalkanen, wie z. B. Diazabicyclooctan (DABCO), Diazabicycloundecen (DBU), Diazabicyclononan (DBN) tertiären Aminen wie Trimethylamin, Triethylamin, Triisopropylamin, Diisopropylethylamin oder Tripropylamin, N,N-Dimethylpiperazin, N-Methylpyridin, N-Methylpyrrolidon, Quinuclidin und dergleichen. Vorzugsweise wird DBU eingesetzt.
3. Verfahren nach einer der vorhergehenden Ausführungsformen, wobei die oxidative Decarbonylierung in einem organischen Lösungsmittel erfolgt, worin der Katalysator gelöst ist.
   Als nicht limitierende Beispiele für organische Lösungsmittel sind zu nennen: Polare, a-protische Lösungsmittel wie Dimethylformamid (DMF), Hexamethylphosphoramid (HMPA), Dimethylsulfoxid (DMSO), Tetramethylharnstoff und Dimethylacetamid, sowie Mischungen davon. Weitere geeignete organische Lösungsmittel, welche alleine oder in Kombination mit obigen aprotischen organischen Lösungsmitteln einsetzbar sind, sind Alkanole, wie beispielsweise Methanol, Ethanol, Propanol oder Butanole, wie *tert*-Butanol sowie Tetrahydrofuran, Dioxan oder Benzol. Vorzugsweise wird DMF eingesetzt.
4. Verfahren nach einer der vorhergehenden Ausführungsformen, wobei man den Katalysator in situ bildet, indem man zu einem Cu-(II)-Salz einen insbesondere zweizähnigen Liganden, vorzugsweise Diamin-Liganden, gibt.
   Das erfindungsgemäß eingesetzte Cu-(II)-Salz ist beispielsweise ausgewählt unter Cu-(II)-acetat, -formiat, -sulfat, -chlorid oder-nitrat, Vorzugsweise wird Cu(OAc)₂ eingesetzt.
   Geeignete Komplexliganden sind insbesondere zweizähnige Kupfer-komplexierende Amin-Liganden, wie *N*,*N*,*N*',*N*'-Tetramethylethylendiamin (TMEDA), 1,10-Phenantrolin und 2,2-Bipyridyl. Vorzugsweise wird TMEDA eingesetzt.
5. Verfahren nach einer der vorhergehenden Ausführungsformen, wobei die oxidative Decarbonylierung bei einer Temperatur im Bereich von 10 bis 70, insbesondere 30 bis 60 °C, über eine Dauer von 0,1 bis 40, insbesondere 0,10 bis 30h und bei einem Druck von 0,1 bis 10, insbesondere 1 bis 2 atm durchgeführt wird. Eine Durchführung des Verfahrens im Vakuum, wie z.B. bei einem Unterdruck im Bereich von 0,001 bis 0,99 bar, oder insbesondere 0,01 bis 0,5 oder 0,1 bis 0,25 bar ist ebenfalls denkbar.
6. Verfahren nach einer der vorhergehenden Ausführungsformen, wobei in den Verbindungen der Formel **X** und **XI** m für 15 und/oder n für 1, insbesondere m für 15 und n für 1 steht.
7. Verfahren nach einer der vorhergehenden Ausführungsformen, wobei man (E/Z)-Cyclopentadec-8-enyl-1-carbaldehyd oder eine diese Verbindung enthaltendes Stoffgemisch, wie z.B. eine Mischung aus (E/Z)-Cyclopentadec-8-enyl- und (E/Z)-Cyclopentadec-7-enyl-1-carbaldehyd, einsetzt.
8. Verfahren nach Ausführungsform 7, wobei man wenigstens eine Verbindung, ausgewählt unter (E/Z)-Cyclopentadec-8-en-1-on und (E/Z)-Cyclopentadec-7-en-1-on erhält.
9. Verfahren nach Ausführungsform 8, wobei man wenigstens eine Verbindung ausgewählt unter Verbindungen der folgenden Formeln I, **II, III** und **IV** 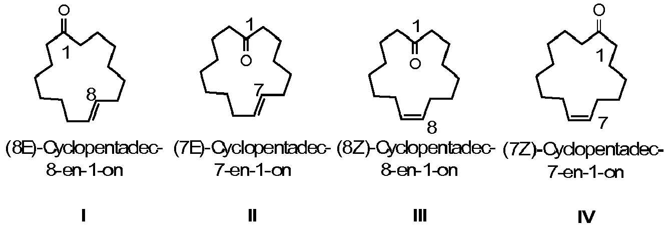 erhält, insbesondere ein Stoffgemisch im Wesentlichen, (d.h. zu wenigstens 80 % oder 90% (FI.-%) aus den Verbindungen **I** und **III** oder ein Stoffgemisch im Wesentlichen (d.h. zu wenigstens 80 % oder 90% (FI.-%) aus den Verbindungen I, **II** und **III** oder aus den Verbindungen **I, II** und **III** und gegebenenfalls **IV.**
10. Verfahren nach einer der vorhergehenden Ausführungsformen, wobei man in Stufe a) ein Reaktionsprodukt einsetzt, das man erhält aus der Distickstoffmonoxid-Oxidation einer cycloaliphatische Verbindung der Formel **XII** 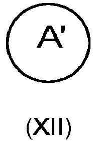 worin A' für einen cycloaliphatischen Kohlenwasserstoffrest mit m+1 Ringkohlenstoffatomen steht, wobei m für einen ganzzahligen Wert von 13 bis 17, wie z.B. 13, 14, 15, 16 oder 17, insbesondere 15, steht, und gegebenenfalls n+1 C=C-Doppelbindungen aufweist, und worin n für einen ganzzahligen Wert von 1, 2 oder 3, insbesondere 1 steht,
11. Verfahren nach Ausführungsform 10, worin in der Verbindung der Formel **XII** m für 15 und n für 1 steht.
12. Verfahren nach Ausführungsform 11, worin die Verbindung der Formel **XII** Cyclohexadeca-1,9-dien ist.
13. Verfahren zur Herstellung von Globanon ((E/Z)-Cyclohexadec-8-en-1on), wobei man
   a) Cyclohexadeca-1,9-dien mit Distickstoffmonoxid oxidiert, wobei man ein Reaktionsgemisch erhält, welches (E/Z)-Cyclohexadec-8-en-1on im Gemisch wenigstens eine Verbindung ausgewählt unter (E/Z)-Cyclopentadec-8-enylcarbaldehyd und (E/Z)-Cyclopentadec-7-enylcarbaldehyd (insbesondere die E-Form) enthält; wobei man nichtumgesetztes Cyclohexadeca-1,9-dien gegebenenfalls abtrennt, z.B. destillativ, und dieses dann gegebenenfalls in das Verfahren, Stufe a), zurückführt;
   b) das Reaktionsgemisch aus Stufe a) einer oxidativen Decarbonylierungsreaktion nach einer der Ausführungsformen 1 bis 5 unterzieht; und
   c) Globanon von den dabei gebildeten Cyclopentadecenonen **I - IV** trennt.
14. Verfahren nach Ausführungsform 13, wobei die Trennung in Stufe c) destillativ erfolgt.
15. Stoff oder Stoffgemisch enthaltend wenigstens eine makrocyclische Ketoverbindung der obigen allgemeinen Formel **X.**
16. Stoffgemisch nach Ausführungsform 15, umfassend im Wesentlichen (d.h. zu wenigstens 80% oder 90% (FI.-%) wenigstens zwei Verbindungen, ausgewählt unter (E/Z)-Cyclopentadec-8-en-1-on und (E/Z)-Cyclopentadec-7-en-1-on.
17. Stoffgemisch nach Ausführungsform 16, umfassend im Wesentlichen (d.h. zu wenigstens 80% oder 90% (FI.-%) wenigstens zwei Verbindungen ausgewählt unter Verbindungen der folgenden Formeln **I, II** und **III** 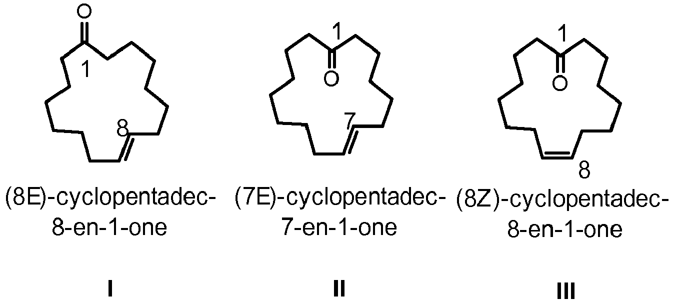 oder umfassend im Wesentlichen (d.h. zu wenigstens 80% oder 90% (FI.-%) wenigstens zwei Verbindungen ausgewählt unter Verbindungen der folgenden Formeln I, II, III und IV 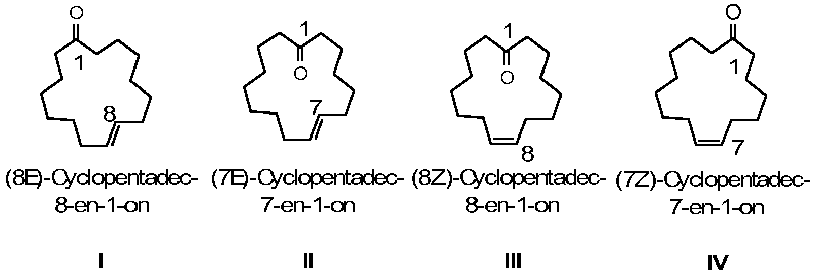
18. Verwendung wenigstens eines Stoffes oder Stoffgemisches nach einer der Ausführungsformen 15 bis 17 als Aromachemikalie, insbesondere als Riechstoff.
19. Verwendung nach Ausführungsform 18 in Mitteln, ausgewählt unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.
20. Verwendung nach einer der Ausführungsformen 18 und 19 eines Gemischs, im Wesentlichen enthaltend (E)-Cyclopentadec-8-en-1-on (**I**) und (Z)-Cyclopentadec-8-en-1-on (**III**).
21. Verwendung nach Ausführungsform 20, wobei das Molverhältnis von (**I**) zu (**III**) im Bereich von etwa 1:1 bis etwa 10:1, insbesondere 1:1 bis 5:1liegt
22. Verwendung nach einer der Ausführungsformen 18 bis 21 zur Erzeugung einer Moschusnote in einer Riechstoffzusammensetzung.
23. Verwendung nach Ausführungsform 22, zum Vermitteln, Modifizieren und/oder Verstärken einer Moschusduftnote in einer Riechstoffzusammensetzung durch Beimischen einer sensorisch wirksamen Menge wenigstens eines Stoffes oder ein Stoffgemisches gemäß der Definition in einem der Ausführungsformen 15 bis 17.
24. Riechstoffzusammensetzung, enthaltend wenigstens einen Stoff oder ein Stoffgemisch gemäß der Definition in einer der Ausführungsformen 15 bis 17 oder hergestellt nach einem der Verfahren gemäß den Ausführungsformen 1 bis 12.
25. Zusammensetzung nach Ausführungsform 21, enthaltend den Stoff oder das Stoffgemisch in einem Gewichtsanteil von 0,01 bis 99,9 Gew.-%, 1 bis 80 Gew.-%, 2 bis 50 Gew.-%, 3 bis 25 oder 5 bis 15 Gew.-% bezogen auf das Gesamtgewicht der Zusammensetzung.
26. Mittel enthaltend wenigstens einen Stoff oder ein Stoffgemisch gemäß der Definition in einer der Ausführungsformen 15 bis 17 oder hergestellt nach einem der Verfahren gemäß den Ausführungsformen 1 bis 12.
27. Mittel nach Ausführungsform 26, enthaltend den Stoff oder das Stoffgemisch in einem Gewichtsanteil von 0,01 bis 99,9 Gew.-%, 1 bis 80 Gew.-%, 2 bis 50 Gew.-%, 3 bis 25 oder 5 bis 15 Gew.-%, bezogen auf das Gesamtgewicht des Mittels.
28. Mittel nach Ausführungsform 26 oder 27, ausgewählt unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.

### c) Weiter Ausgestaltungen der Erfindung

### c1) Riechstoffzusammensetzungen:

Einem weiteren Aspekt zufolge werden die erfindungsgemäß verwendeten Riechstoffe insbesondere zwecks effizienterer Handhabung und Dosierung, auch als Riechstoffmischungen mit Verdünnungs- oder Lösungsmitteln eingesetzt. Hierbei wird der Anteil der Riechstoffe, bezogen auf die Summe von Riechstoffen und Lösungsmittel, in Gew.-% angegeben.

### Lösungsmittel:

Ein "Lösungsmittel" dient im Sinne der vorliegenden Erfindung der Verdünnung der erfindungsgemäß zu verwendenden Riechstoffe oder der erfindungsgemäßen Riechstoffzusammensetzung, ohne eigene riechende Eigenschaften zu besitzen. Manche Lösungsmittel haben zugleich fixierende Eigenschaften.

Die erfindungsgemäße Verbindung der Formel **X** oder ein oben definiertes Stoffgemisch aus mehreren Verbindungen/Isomeren der Formel **X** kann zu 0,1 bis 99 Gew-% einem Verdünnungs- oder Lösungsmittel beigemischt werden. Bevorzugt sind mindestens 40 Gew.-%ige Lösungen, weiter bevorzugt mindestens 50 Gew.-%ige Lösungen, weiterhin bevorzugt mindestens 60 Gew.-%ige Lösungen, weiter bevorzugt mindestens 70 Gew.-%ige Lösungen, insbesondere bevorzugt mindestens 80 Gew.-%ige Lösungen, weiterhin insbesondere bevorzugt mindestens 90 Gew.-%ige Lösungen, vorzugsweise in olfaktorisch akzeptablen Lösungsmitteln.

Bevorzugte olfaktorisch akzeptable Lösungsmittel sind Ethanol, Isopropanol, Dipropylenglycol (DPG), Propylenglycol, 1,2-Butylenglycol, Glycerin, Diethylenglycolmonoethylether, Diethylphthalat (DEP), Isopropylmyristat (IPM), Triethylcitrat (TEC), Benzylbenzoat (BB) und Benzylacetat. Hierbei wiederum bevorzugt sind Ethanol, Diethylphthalat, Propylenglycol, Dipropylenglycol, Triethylcitrat, Benzylbenzoat und Isopropylmyristat.

Eine "Riechstoffzusammensetzung" ist im Sinne der vorliegenden Erfindung eine Mischung, die neben einer erfindungsgemäßen Verbindung der Formel **X** oder einem oben definierten Stoffgemisch aus mehreren Verbindungen/Isomeren der Formel **X** mindestens einen weiteren Riechstoff umfasst. Insbesondere kann es sich bei einer solchen Riechstoffkomposition um eine Parfümkomposition (ein Parfümöl) handeln.

Erfindungsgemäße Riechstoffkompositionen enthalten, bezogen auf die Gesamtmenge der Riechstoffkomposition, z.B. eine Menge von einer erfindungsgemäßen Verbindung der Formel **X** oder eines oben definierten Stoffgemischs aus mehreren Verbindungen/Isomeren der Formel **X** von 0,01 bis 65 Gew.-%, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%. Das Gewichtsverhältnis von erfindungsgemäßer Verbindung/erfindungsgemäßen Verbindungen der Formel **X** zu der Gesamtmenge an weiteren Riechstoffen liegt z.B. im Bereich von 1:1000 bis 1:0,5 , vorzugsweise im Bereich von 1:700 bis 1:1, besonders bevorzugt im Bereich von 1:500 bis 1:10.

Erfindungsgemäße Riechstoffkompositionen enthalten, bezogen auf die Gesamtmenge der Riechstoffkomposition, z.B. eine Menge von erfindungsgemäßer Verbindung/ erfindungsgemäßen Verbindungen der Formel **X** von 0,01 bis 65 Gew.-% und, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%. Das Gewichtsverhältnis von erfindungsgemäßer Verbindung/erfindungsgemäßen Verbindungen der Formel **X** zu der Gesamtmenge an weiteren (davon verschiedenen) Riechstoffen liegt z.B. im Bereich von 1:1000 bis 1:0,5 , vorzugsweise im Bereich von 1:700 bis 1:1, besonders bevorzugt im Bereich von 1:500 bis 1:10.

Obige Angaben zum Gehalt an Verbindungen der Formel **X** gelten sinngemäß auch für die bevorzugten Verbindungen der Formeln I, **II, III** und **IV,** insbesondere auch für Stoffgemische der Verbindungen der Formeln **I, II, III** und **IV.**

### Weitere Riechstoffe:

Erfindungsgemäße Riechstoffkompositionen enthalten neben der erfindungsgemäßen Verbindung/ den erfindungsgemäßen Verbindungen der Formel **X** (insbesondere der Formeln **I, II** und **III)** zumindest einen weiteren Riechstoff, vorzugsweise 2, 3, 4, 5, 6, 7, 8 oder mehr weitere Riechstoffe, wobei weitere Riechstoffe z.B. ausgewählt sind unter:

Alpha-Hexylzimtaldehyd, 2-Phenoxyethylisobutyrat (Phenirat¹), Dihydromyrcenol (2,6-Dimethyl-7-octen-2-ol), Methyldihydrojasmonat (vorzugsweise mit einem Gehalt an *cis*-Isomerem von mehr als 60 Gew.-%) (Hedione⁹, Hedione HC⁹), 4,6,6,7,8,8-Hexamethyl-1,3,4,6,7,8-hexahydro-cyclopenta[g]benzopyran (Galaxolid³), Tetrahydrolinalool (3,7-Dimethyloctan-3-ol), Ethyllinalool, Benzylsalicylat, 2-Methyl-3-(4-*tert*-butylphenyl)propanal (Lilial²), Zimtalkohol, 4,7-Methano-3a,4,5,6,7,7a-hexahydro-5-indenylacetat und/oder 4,7-Methano-3a,4,5,6,7,7a-hexahydro-6-indenylacetat (Herbaflorat¹), Citronellol, Citronellylacetat, Tetrahydrogeraniol, Vanillin, Linalylacetat, Styrolylacetat (1-Phenylethylacetat), Octahydro-2,3,8,8-tetramethyl-2-acetonaphthon und/oder 2-Acetyl-1,2,3,4,6,7,8-octahydro-2,3,8,8-tetramethyl-naphtalin (Iso E Super³), Hexylsalicylat, 4-*tert*-Butylcyclohexylacetat (Oryclone¹), 2-*tert*-Butylcyclohexylacetat (Agrumex HC¹), Alphalonon (4-(2,2,6-Trimethyl-2-cyclohexen-1-yl)-3-buten-2-on), n-alpha-Methylionon, alpha-Isomethylionon, Coumarin, Terpinylacetat, 2-Phenylethylalkohol, 4-(4-Hydroxy-4-methylpentyl)-3-cyclohexencarboxaldehyd (Lyral³), alpha-Amylzimtaldehyd, Ethylenbrassylat, (E)- und/oder (Z)-3-Methylcyclopentadec-5-enon (Muscenon⁹), 15-Pentadec-11-enolid und/oder 15-Pentadec-12-enolid (Globalide¹), 15-Cyclopentadecanolid (Macrolide¹), 1-(5,6,7,8-Tetrahydro-3,5,5,6,8,8-hexamethyl-2-naphthalenyl)ethanon (Tonalid¹⁰), 2-Isobutyl-4-methyltetrahydro-2H-pyran-4-ol (Florol⁹), 2-Ethyl-4-(2,2,3-trimethyl-3-cyclopenten-1-yl)-2-buten-1-ol (Sandolen¹), *cis*-3-Hexenyl-acetat, *trans*-3-Hexenylacetat, *trans*-2,-*cis*-6-Nonadienol, 2,4-Dimethyl-3-cyclohexencarboxaldehyd (Vertocitral¹), 2,4,4,7-Tetramethyl-oct-6-en-3-on (Claritone¹), 2,6-Dimethyl-5-hepten-1-al (Melonal²), Borneol, 3-(3-Isopropylphenyl)butanal (Florhydral²), 2-Methyl-3-(3,4-methylendioxyphenyl)propanal (Helional³), 3-(4-Ethylphenyl)-2,2-dimethylpropanal (Florazon¹), 7-Methyl-2H-1,5-benzodioxepin-3(4H)-on (Calone¹⁹⁵¹⁵), 3,3,5-Trimethylcyclohexylacetat (vorzugsweise mit einem Gehalt an *cis*-Isomeren von 70 Gew.-%) oder mehr und 2,5,5-Trimethyl-1,2,3,4,4a,5,6,7-octahydronaphthalin-2-ol (Ambrinol S¹). Die vorangehend genannten Riechstoffe werden im Rahmen der vorliegenden Erfindung demnach bevorzugt mit erfindungsgemäßen Mischungen kombiniert.

Sofern vorstehend Handelsnamen angegeben sind, beziehen sich diese auf folgende Quellen:
¹ Handelsname Symrise GmbH, Deutschland;
² Handelsname Givaudan AG, Schweiz;
³ Handelsname International Flavors & Fragrances Inc., USA;
⁵ Handelsname Danisco Seillans S.A., Frankreich;
⁹ Handelsname Firmenich S.A., Schweiz;
¹⁰ Handelsname PFW Aroma Chemicals B.V., Niederlande.

Weitere Riechstoffe, mit denen (E/Z)-Cyclopentadec-7/8-enon z.B. zu einer Riechstoffkomposition kombiniert werden können, finden sich z.B. in S. Arctander, Perfume and Flavor Chemicals, Vol. I und II, Montclair, N. J., 1969, Selbstverlag oder K. Bauer, D. Garbe und H. Surburg, Common Fragrance and Flavor Materials, 4rd. Ed., Wiley- VCH, Weinheim 2001. Im Einzelnen seien genannt:
Extrakte aus natürlichen Rohstoffen wie etherischen Ölen, Concretes, Absolues, Resine, Resinoide, Balsame, Tinkturen wie z.B.
Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptuscitriodora Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzelriechstoffe aus der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methylen-heptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyl-oxyacetaldehyd; (E/Z)-1-(1-Methoxy-propoxy)--3-hexen; der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäure-nitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; (E/Z)-Ethyl2,4-decadienoat; Methyl-2-octinat; Methyl-2-noninat; Allyl-2-isoamyloxyacetat; Methyl-3,7-dimethyl-2,6-octadienoat; 4-Methyl-2-pentyl-crotonat;
der acyclischen Terpenalkohole wie z.B. Geraniol; Nerol; Linalool; Lavadulol; Nerolidol; Farnesol; Tetrahydrolinalool; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,1 -Trimethyl-9-undecenal; Geranylaceton; sowie die Dimethyl- und Diethylacetale von Geranial, Neral, 7-Hydroxy-3,7-dimethyloctanal; der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-buten-al; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-*tert*-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyl-tetrahydro-2H-pyran-4-ol;
der cycloaliphatischen Alkohole wie z.B. alpha-3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-rimethyl-3-cyclopent-1-yl)-2-buten-1-ol; 2-Ethyl-4-(2,2,3-Trimethyl-3-cyclopent-1-yl)-2-buten-1-ol; 3-Methyl-5-(2,2,3-Trimethyl-3-cyclopent-1-yl)-pentan-2-ol; 3-Methyl-5-(2,2,3-Trimethyl-3-cyclopent-1-yl)-4-penten-2-ol; 3,3-Dimethyl-5-(2,2,3-Trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydro-naphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-*tert*-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; *cis*-3-Methylpent-2-en-1-yl-cyclopent-2-en-1-on; 3-Methyl-2-pentyl-2-cyclopenten-1-on; 3-Methyl-4-cyclopenta-decenon; 3-Methyl-5-cyclopentadecenon; 3-Methylcyclopentadecanon; 4-(1-Ethoxyvinyl)-3,3,5,5-tetramethylcyclohexanon; 4-*tert*-Pentylcyclohexanon; Cyclohexadec-5-en-1-on; 6,7-Dihydro-1,1,2,3,3-pentamethyl-4(5H)-indanon; 8-Cyclohexadecen-1-on; 7-Cyclohexadecen-1-on; (7/8)-Cyclohexadecen-1-on; 9-Cyclohepta-decen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-Trimethylcyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z.B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; *tert*-Butyl-(2,4-dimethyl-3-cyclohexen-1-yl)keton;
der Ester cyclischer Alkohole wie z.B. 2-*tert*-Butylcyclohexylacetat; 4-*tert*-Butylcyclohexylacetat; 2-*tert*-Pentylcyclohexylacetat; 4-*tert*-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5 bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z.B. Allyl-3-cyclohexylpropionat; Allylcyclohexyloxyacetat; *cis-* und *trans*-Methyldihydrojasmonat; *cis-* und *trans*-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol, 2-Phenylethylalkohol, 3-Phenylpropanol; 2-Phenylpropanol; 2-Phenoxyethanol; 2,2-Dimethyl-3-phenylpropanol; 2,2-Dimethyl-3-(3-methylphenyl)-propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropyl-phenyl)-ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-*tert-*butylphenyl)propanal; 2-Methyl-3-(4-isobutylphenyl)propanal; 3-(4-*tert*-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4 Methylacetophenon; 4-Methoxyacetophenon; 4-*tert*-Butyl-2,6-dimethylacetophenon; 4-Phenyl-2-butanon; 4-(4-Hydroxyphenyl)-2-butanon; 1-(2-Naphthalenyl)ethanon; 2-Benzofuranylethanon;(3-Methyl-2-benzofuranyl)ethanon; Benzophenon; 1,1,2,3,3,6-Hexamethyl-5-indanylmethylketon; 6-*tert-*Butyl-1,1-dimethyl-4-indanylmethylketon; 1-[2,3-Dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; *cis*-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-*tert*-butylacetophenon; Zimtsäurenitril; 3-Methyl-5-phenyl-2-pentensäurenitril; 3-Methyl-5-phenylpentansäurenitril; Methylanthranilat; Methyl-N-methylanthranilat; Schiff''sche Basen von Methylanthranilat mit 7-Hydroxy-3,7-dimethyloctanal, 2-Methyl-3-(4-*tert*-butylphenyl)propanal oder 2,4-Dimethyl-3-cyclohexencarbaldehyd; 6-Isopropylchinolin; 6-Isobutylchinolin; 6-sec-Butylchinolin; 2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; *cis-* und *trans*-11-Pentadecen-1,15-olid; *cis-* und *trans-*12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

### c2) Riechstoffhaltige Artikel

Erfindungsgemäßes (E/Z)-Cyclopentadec-7/8-enon oder erfindungsgemäße Riechstoffkompositionen können in eine Reihe von Produkten eingearbeitet bzw. auf solche Produkte appliziert werden.

Erfindungsgemäße Riechstoffe können bei der Herstellung parfümierter Artikel eingesetzt. Die olfaktorischen Eigenschaften ebenso wie die stofflichen Eigenschaften (wie Löslichkeit in gängigen Lösungsmitteln und Kompatibilität mit gängigen weiteren Bestandteilen derartiger Produkte) sowie die toxikologische Unbedenklichkeit der erfindungsgemäßen Riechstoffe unterstreichen ihre besondere Eignung für die genannten Einsatzzwecke. Die positiven Eigenschaften tragen dazu bei, dass die erfindungsgemäß verwendeten Riechstoffe und die erfindungsgemäßen Riechstoffkompositionen besonders bevorzugt in Parfümerzeugnissen, Körperpflegeprodukten, Hygieneartikeln, Textilwaschmittel sowie in Reinigungsmitteln für feste Oberflächen eingesetzt werden.

Der parfümierte Artikel ist z.B. ausgewählt unter Parfümerzeugnissen, Körperpflegeprodukten, Hygieneartikeln, Textilwaschmitteln und Reinigungsmitteln für feste Oberflächen. Bevorzugte erfindungsgemäße parfümierte Artikel sind weiterhin ausgewählt unter:
Parfümerzeugnissen, ausgewählt unter Parfüm-Extrakten, Eau de Parfums, Eau de Toilettes, Eau de Colognes, Eau de Solide, Extrait Parfum, Luftverbesserern in flüssiger, gelartiger oder auf einem festen Träger aufgebrachter Form, Aerosolsprays, Duftreinigern und -ölen;
Körperpflegeprodukten, ausgewählt unter Rasierwässern, Pre-shave-Produkten, Splash-Colognes, festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und - lotionen, Bräunungscremes und -lotionen, Haarpflegeprodukten wie z.B. Haarsprays, Haargelen, festigende Haarlotionen, Haarspülungen, Haarshampoo, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara, Zahnpasta, Zahnseide;
Hygieneartikeln, ausgewählt unter Kerzen, Lampenölen, Räucherstäbchen, Insektiziden, Repellentien, Treibstoffen, Rostentfernern, parfümierten Erfrischungstüchern, Achselpads, Babywindeln, Damenbinden, Toilettenpapier, Kosmetiktüchern, Taschentüchern, Spülmaschinendeo;
Reinigungsmitteln für feste Oberflächen, ausgewählt unter parfümierten sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Wachsen und Polituren wie Möbelpolituren, Fußbodenwachsen, Schuhcremes, Desinfektionsmitteln, Oberflächendesinfektionsmitteln und Sanitärreinigern, Bremsenreinigern, Rohrreinigern, Entkalkern, Grill- und Backofenreinigern, Algen- und Moosentfernern, Schimmelentfernern, Fassadenreinigungsmitteln;
Textilwaschmitteln, ausgewählt unter flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten.

Einem weiteren Aspekt zufolge sind die erfindungsgemäß verwendeten Riechstoffe und die erfindungsgemäßen Riechstoffkompositionen für den Einsatz in tensidhaltigen parfümierten Artikeln geeignet. Gesucht werden nämlich - insbesondere für die Parfümierung von tensidhaltigen Formulierungen wie zum Beispiel Reinigungsmitteln (insbesondere Geschirrspülmittel und Allzweckreiniger) - häufig Riechstoffe und/oder Riechstoffkompositionen mit einer Rosenkopfnote und ausgeprägter Natürlichkeit.

Einem weiteren Aspekt zufolge können erfindungsgemäß verwendete Riechstoffe und erfindungsgemäße Riechstoffkompositionen als Mittel zum Versehen von (a) Haaren oder (b) textilen Fasern mit der Geruchsnote rosig verwendet werden.

Die erfindungsgemäß zu verwendenden Riechstoffe und erfindungsgemäße Riechstoffkompositionen eignen sich daher besonders gut für den Einsatz in tensidhaltigen parfümierten Artikeln.

Bevorzugt ist es, wenn der parfümierte Artikel einer der folgenden ist:
- ein saures, alkalisches oder neutrales Reinigungsmittel, das insbesondere aus der Gruppe ausgewählt ist bestehend aus Allzweckreinigern, Fußbodenreinigern, Fensterglasreinigern, Geschirrspülmitteln, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektions-mitteln, Oberflächendesinfektionsmitteln,
- ein Luftverbesserer in flüssiger, gelartiger oder auf einem festen Träger aufgebrachter Form oder als Aerosolspray,
- ein Wachs oder eine Politur, die insbesondere aus der Gruppe ausgewählt ist bestehend aus Möbelpolituren, Fußbodenwachsen und Schuhcremes, oder
- ein Körperpflegemittel, das insbesondere aus der Gruppe ausgewählt ist bestehend aus Duschgelen und Shampoos Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Rollons, Deosticks, Deocremes, Produkten der dekorativen Kosmetik.

Inhaltstoffe, mit denen erfindungsgemäß verwendete Riechstoffe oder erfindungsgemäße Riechstoffkompositionen vorzugsweise kombiniert werden können, sind beispielsweise: Konservierungsmittel, Abrasiva, Antiaknemittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, Schweiss hemmende Mittel, antiseptische Mittel, Antistatika, Binder, Puffer, Trägermaterialien, Chelatbildner, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, a-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Einem weiteren Aspekt zufolge verwendet man die Riechstoffe bei der Herstellung der parfümierten Artikel in flüssiger Form, unverdünnt oder mit einem Lösungsmittel verdünnt oder in Form einer Riechstoffkomposition. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw. Für die genannten Lösungsmittel gilt, dass diese, im Falle des Vorhandenseins eigener olfaktorischer Eigenschaften, ausschließlich dem Bestandteil "Lösungsmittel" und nicht den "Riechstoffen" zuzuordnen sind.

Die in den erfindungsgemäßen parfümierten Artikeln enthaltenen Riechstoffe und/oder Riechstoffkompositionen können dabei in einer Ausführungsform an einem Trägerstoff absorbiert sein, der sowohl für eine feine Verteilung des Riechstoffs oder der Riechstoffkomposition im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die erfindungsgemäß verwendeten Riechstoffe und die erfindungsgemäßen Riechstoffkompositionen können auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden. Die Eigenschaften können durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung kann beispielsweise durch das sogenannte Koazervationsverfahren mit Hilfe von Kapselmaterialien, z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschlusskomplexe können z.B. durch Eintragen von Dispersionen von Riechstoffkompositionen und Cyclodextrinen oder Harnstoffderivaten in ein geeignetes Lösungsmittel, z.B. Wasser, hergestellt werden. Extrusionsprodukte können durch Verschmelzen erfindungsgemäß verwendeter Riechstoffe und erfindungsgemäßer Riechstoffkompositionen mit einem geeigneten wachsartigen Stoff und durch Extrusion mit nachfolgender Erstarrung, ggf. in einem geeigneten Lösungsmittel, z.B. Isopropanol, hergestellt werden.

### c3) Herstellung erfindungsgemäßer Riechstoffe

Die erfindungsgemäßen makrocyclischen Ketone der Formel **X** werden ausgehend von den korrespondierenden Carbaldehyden der Formel **XI** herstellt 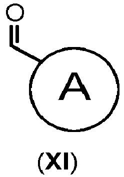 worin A für einen cycloaliphatischen Kohlenwasserstoffrest mit m Ringkohlenstoffatomen steht, wobei m für einen ganzzahligen Wert von 13 bis 17 steht, und gegebenenfalls n C=C-Doppelbindungen aufweist, wobei n für einen ganzzahligen Wert von 1, 2 oder 3 steht.

Die Carbaldehyde ihrerseits sind beispielsweise ausgehend von den korrespondierenden cycloaliphatischen Verbindungen der Formel **XII** 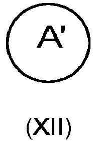 worin A' für einen cycloaliphatischen Kohlenwasserstoffrest mit m+1 Ringkohlenstoffatomen steht, wobei m für einen ganzzahligen Wert von 13 bis 17 steht, und gegebenenfalls n+1 C=C-Doppelbindungen aufweist, und worin n für einen ganzzahligen Wert von 1, 2 oder 3 steht und durch Distickstoffmonoxid-Oxidation zugänglich ist.

### i) Synthese des Carbaldehyd (XI) durch N₂O-Oxidation

Wie z.B. beschrieben in der PCT/EP2015/072544 der Anmelderin.

Ausgehend vom korrespondierenden aliphatischen Carbocyclus, insbesondere einen cyclischen einfach oder mehrfach ungesättigten Olefin **XII** (d.h. im Vergleich zum cyclischen Rest A im Produkt der Formel **X** enthält die Ausgangsverbindung **XII** eine zusätzliche C=C-Doppelbindung und ein zusätzliches Ringkohlenstoffatom) sind Verbindungen der Formel **XI** durch Distickstoffmonoxid Oxidation zugänglich, wie z.B. beschrieben in der WO 2012/084673.

Beispiel für eine geeignete Ausgangsverbindung (die sowohl in stereoisomerenreiner Form als auch in Form von Stereoisomerengemischen eingesetzt werden kann) zur Herstellung von Verbindungen der Formel **XI,** worin A für einen einfach ungesättigten C₁₅-Rest steht, ist Cyclohexa-deca-1,9-dien das entweder käuflich erhältlich ist oder gemäß Beispiel 2 der EP-A-1 288 181) hergestellt werden kann.

Insbesondere wird dabei ein cyclisches Olefin durch Umsetzung mit Distickstoffmonoxid oxidiert. Distickstoffmonoxid kann dabei rein oder ggf. im Gemisch mit anderen bei Reaktionsbedingungen gasförmigen Substanzen, wie z.B. Kohlenstoffdioxid zur Verdünnung eingesetzt werden.

Dabei kann für die Umsetzung des cyclischen Olefins mit Distickstoffmonoxid in Substanz oder in Gegenwart mindestens eines geeigneten Lösungsmittels oder Verdünnungsmittels erfolgen. Vorzugsweise erfolgt die Umsetzung in Substanz. Im Wesentlichen alle gängigen Lösungs- und/oder Verdünnungsmittel sind hierbei geeignet, jedoch unter der Maßgabe, dass sie weder eine C-C-Doppelbindung, noch eine C-C-Dreifachbindung, noch eine Aldehydgruppe aufweisen. Als geeignete Lösungsmittel sind unter anderem zu nennen: cyclische Alkane, beispielsweise Cyclohexan, Cyclopentan, Cyclooctan, Cyclododecan oder gesättigte aliphatische oder aromatische, gegebenenfalls alkylsubstituierte Kohlenwasserstoffe zu nennen.

Die Temperatur bei der Umsetzung liegt z.B. bei 140 bis 350°C, wie insbesondere bei 180 bis 320 °C oder bei 200 bis 300 °C. Es ist auch möglich, die Umsetzung bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden. Insbesondere ist die Umsetzungstemperatur aber im Wesentlichen konstant. Die Reaktion kann aber auch vorzugsweise adiabatisch durchgeführt werden, so dass im Reaktor die Temperatur steigt.

Der Druck bei der Umsetzung des cyclischen Olefins mit Distickstoffmonoxid liegt insbesondere höher als der Eigendruck des Edukt bzw. Produktgemisches bei der gewählten Reaktionstemperatur oder den gewählten Reaktionstemperaturen. Der Druck liegt z.B. bei 1 bis 1000 bar, wie z.B. bei 40 bis 300 bar oder bei 50 bis 200 bar.

Es ist möglich, die Umsetzung des cyclischen Olefins mit Distickstoffmonoxid bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden. Insbesondere ist Druck während der Umsetzung aber im Wesentlichen konstant.

Hinsichtlich der für die Umsetzung (im Labor- oder Produktionsmaßstab) einsetzbaren Reaktoren gibt es keine besonderen Beschränkungen. Insbesondere kann die Umsetzung in Batch-Fahrweise oder in kontinuierlicher Fahrweise erfolgen. Folglich können beispielsweise als Reaktoren mindestens ein CSTR (Continuous Stirred Tank Reactor) mit mindestens einem internen und/oder mindestens einem externen Wärmetauscher, mindestens ein Rohrreaktor, mindestens ein Rohrbündelreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden. Insbesondere wird die Umsetzung mit Distickstoffmonoxid in einem einzigen Reaktor durchgeführt. Beispielsweise kann die Umsetzung in kontinuierlicher Fahrweise oder in Batch-Fahrweise erfolgen.

Die Verweilzeit der Reaktionsmischung im Reaktor liegt im Allgemeinen im Bereich von 0,1 bis 40 Stunden, bevorzugt im Bereich von 0,1 bis 30 Stunden, weiter bevorzugt im Bereich von 2 bis 7 Stunden.

Im Feed liegt das Molverhältnis von Distickstoffmonoxid und dem cyclischen Olefin im Allgemeinen im Bereich von 0,01 bis 30, wie z.B. im Bereich von 0,03 bis10, besonders bevorzugt im Bereich von 0,05 bis 1 und ganz besonders bevorzugt im Bereich von 0,08 bis 0,2.

Da Distickstoffmonoxid bevorzugt im Unterschuss eingesetzt wird, wird nur ein Teil des Olefins **XII** (z.B.Cyclohexadeca-1,9-dien) umgesetzt. Nicht umgesetztes Olefin (z.B.Cyclohexadeca-1,9-dien) wird destillativ vom Reaktionsprodukt getrennt und der Reaktion wieder zugeführt. Dabei fällt das nicht umgesetzte Olefin, (z.B. Cyclohexadeca-1,9-dien) als Kopfprodukt und das Reaktionsprodukt als Sumpfprodukt der Kolonne an. Die Destillation findet hierbei z.B. bei 20 mbar Kopfdruck und z.B. 210 °C Sumpftemperatur statt. Der Differenzdruck über die Kolonne beträgt z.B. 18 mbar. Die Kolonne wird z.B. mit einer strukturierten Gewebepackung vom Typ Montz A3 ausgestattet. Die Packungshöhe beträgt dabei z.B. 4 m und der Zulauf liegt z.B. bei 2 m. Aus dem erhaltenen Sumpfaustrag wird anschließend die Verbindung **XI** als Nebenkomponente destillativ isoliert.

Das gewünschte Reaktionsprodukt **XI** entsteht dabei als Nebenkomponente, so dass das Reaktionsgemisch in geeigneter Weise gereinigt werden muss.

In einer weiteren Variante der Erfindung erfolgt aber keine weitere Aufreinigung sondern die direkte Umsetzung des Reaktionsgemischs zu gewünschten Keton der Formel **X.** Eine zusätzliche Reinigung kann aber ebenfalls durchgeführt werden.

Im Falle einer Reinigung kann diese z.B. destillativ (wie insbesondere durch fraktionierte Destillation, vorzugsweise bei vermindertem Druck) oder chromatographisch erfolgen. Geeignete Reinigungsmethoden sind dem Fachmann geläufig. Die Reinigung kann z.B. batchwiese als auch kontinuierlich erfolgen.

Beispielsweise kann die Destillation mittels Destillationskolonne mit dem Fachmann bekannten Packungen verwendet. Die optimalen Destillationsbedingungen sind vom Fachmann ohne unzumutbaren Aufwand festlegbar. Die Destillation kann insbesondere im Vakuum, beispielsweise bei einem Druck < 1000 mbar, < 500 mbar, < 300 mbar, < 100 mbar oder < 10 mbar durchgeführt werden. Die verwendete Destillationskolonne kann mehrere, wie z.B. mindestens 20, mindestens 25 oder mindestens 30 theoretische, wie z.B. bis zu 70 Trennstufen aufweisen. Das Rücklaufverhältnis kann z.B. im Bereich von etwa 5 bis 100 liegen und mindestens 20, mindestens 25 oder mindestens 30 betragen und beträgt insbesondere etwa 100 für eine besonders vorteilhafte Fraktionierung.

Beispielsweise kann auch eine Säulenchromatographie an Stelle oder im Anschluss an eine destillative Reinigung erfolgen. Hierzu verwendet man dem Fachmann bekannte Säulenmaterialen und Laufmittel. Die optimalen Chromatographiebedingungen, wie Säulengeometrie und Geschwindigkeit des Laufmittels, sind vom Fachmann ohne unzumutbaren Aufwand festlegbar.

Beispiele für geeignete Säulenmaterialien sind polare Adsorptionsmittel wie z. B. Eisenoxid Fe₂O₃, Aluminiumoxid, Kohlenhydrate oder Kieselgel mit oder ohne Zusätze wie z. B. Fluoreszenzindikatoren oder Gips.

Beispiele für geeignete Laufmittel sind: aliphatische oder aromatische Laufmittel, wie z.B. Alkane oder Cycloalkane, wie z.B. Pentan, Petrolether, Hexan, Heptan, Toluol oder die korrespondierenden cyclischen Verbindungen; aliphatische Ether, Ester oder, wie z.B. Et₂O, MTBE, EtOAc, Aceton oder Mischungen solcher Laufmitteln, wie z. B. Hexan/MTBE, Hexan/EtOAc, Pentan/Et₂O, Petrolether/Et₂O.

Man kann dabei einen gewünschten Carbaldehyd der Formel **X,** oder Gemische davon, in Reinform oder in einer Reinheit von mehr als 20, wie z.B. mehr als 30 mehr als 40, mehr als 50, mehr als 60, mehr als 70 oder mehr als 80 Gew.-%. isolieren.

Der Carbaldehyd der Formel **XI** kann dabei in stereoisomerenreiner Form, oder insbesondere als Gemisch zweier oder mehrerer Stereoisomeren, insbesondere wenn Rest A eine C=C Doppelbindung aufweist, isoliert werden.

Insbesondere sind auf diesem Weg *trans*-Cyclopentadec-8-enyl-1-carbaldehyd und/ oder *cis-*Cyclopentadec-8-enyl-1-carbaldehyd zugänglich.

### ii) Synthese des cyclischen Ketons (X) durch oxidative Decarbonylierung

Ausgehend vom korrespondierenden cyclischen Carbaldehyd der allgemeinen Formel **(XI)** (gereinigt oder als Reaktionsgemisch, wie oben beschrieben, erhältlich), erfolgt die oxidative Decarbonylierung in Anlehnung an die aus dem Stand der Technik bekannten Cu-(II)- basierten Decarbonylierungen (vgl. z.B. a) Tetrahedron Letters 1969, 12, 985; US3496197; b) Tetrahedron Letters 1995, 4641, c) Org. Lett. 2010, 2630, d) Bioorg. Med. Chem. Lett. 2013, 23, 5949, e) Chin. Chem. Lett. 2014, 25, 771.)

Konkretes, nichtlimitierendes Beispiel für eine geeignete Ausgangsverbindung (die sowohl in stereoisomerenreiner Form als auch in Form von Stereoisomerengemischen eingesetzt werden kann) zur Herstellung von Verbindungen der Formel **X,** worin A für einen einfach ungesättigten C₁₅-Rest steht, sind Cyclopentadec-8-enyl und -7-enylcarbaldehyd.

Erfindungsgemäß erfolgt die katalytische Umsetzung des cyclischen Carbaldehyden mit dem molekularen Sauerstoff in Gegenwart mindestens eines geeigneten Lösungsmittels oder Verdünnungsmittels.

Sauerstoff kann dabei als reiner Sauerstoff oder vorzugsweise als Bestandteil von Umgebungsluft oder Magerluft im Prozess verwendet werden.

Als geeignete Lösungsmittel sind unter anderem zu nennen: polare, aprotische Lösungsmittel wie Dimethylformamid (DMF), Hexamethylphosphoramid (HMPA), Dimethylsulfoxid (DMSO), Tetramethylharnstoff und Dimethylacetamid, sowie Mischungen davon. Weitere geeignete organische Lösungsmittel, welche alleine oder in Kombination mit obigen aprotischen organischen Lösungsmitteln einsetzbar sind, sind Alkanole, wie beispielsweise Methanol, Ethanol, Propanol, Isopropanol oder Butanole, wie *tert*-Butanol sowie Tetrahydrofuran, Dioxan oder Benzol.

As Katalysator verwendet man Cu-(II)- basierte, insbesondere homogene Katalysatoren. Diese werden bevorzugt in situ im Reaktionsansatz gebildet, indem man zu einem Cu-(II)-Salz einen insbesondere zweizähnigen Liganden, vorzugsweise Diamin-Liganden, gibt.

Das erfindungsgemäß eingesetzte Cu-(II)-Salz ist beispielsweise ausgewählt unter Cu-(II)-acetat, -formiat, -sulfat, -chlorid oder-nitrat, Vorzugsweise wird Cu(OAc)₂ eingesetzt.

Geeignete Komplexliganden sind insbesondere zweizähnige Kupfer komplexierende Amin-Liganden, wie *N*,*N*,*N*',*N*'-Tetramethylethylendiamin (TMEDA), 1,10-Phenantrolin und 2,2'-Bipyridin. Vorzugsweise wird TMEDA eingesetzt.

Komplexligand und Cu-(II)-Salz werden in etwa äquimolarem Verhältnis eingesetzt. Der molare Anteil an Komplex liegt bei etwa 0,1 bis 10, insbesondre 1 bis 5, vorzugsweise etwa 2-5 mol-%, bezogen auf den eingesetzten Carbaldehyd (**XI**).

Insbesondere wird die Decarbonylierung zusätzlich in Gegenwart einer organischen Base erfolgt. Die im erfindungsgemäßen Verfahren beispielsweise eingesetzte Base ist ausgewählt unter Diazabicycloalkanen, wie z. B. Diazabicyclooctan (DABCO), Diazabicycloundecen (DBU), Diazobicyclononan (DBN), tertiären Aminen wie Trimethylamin, Triethylamin, Diisopropylethylamin oder Tripropylamin, N,N-Dimethylpiperazin, N-Methylpyridin, N-Methylpyrrolidon, Quinuclidin und dergleichen. Vorzugsweise wird DBU eingesetzt. Die Base wird dabei in einem Anteil von 0,1 - 1 equiv, 0,2 - 0,8 equiv oder besonders bevorzugt 0,4 - 0,6 equiv bezogen auf den eingesetzten Carbaldehyden der Formel **(XI)** eingesetzt

Die Temperatur bei der Umsetzung liegt, je nach verwendeten Reaktanden und Lösungsmittel z.B. bei 20 bis 100°C, wie insbesondere bei 30 bis 80 °C insbesondere bei 40 bis 60 °C. Es ist auch möglich, die Umsetzung bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden. Insbesondere ist die Umsetzungstemperatur aber im Wesentlichen konstant.

Der Druck bei der Umsetzung des Carbaldehyden **(XI)** mit Sauerstoff liegt insbesondere bei Umgebungsdruck oder etwa im Bereich des Eigendrucks des Edukt bzw. Produktgemisches bei der gewählten Reaktionstemperatur oder den gewählten Reaktionstemperaturen. Der Druck liegt z.B. bei 0,1 bis 5 bar, wie z.B. bei 0,1 bis 3 bar vorzugsweise etwa 1 bar.

Es ist möglich, die Umsetzung bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden. Insbesondere ist Druck während der Umsetzung aber im Wesentlichen konstant.

Insbesondere kann die Umsetzung in Batch-Fahrweise oder in kontinuierlicher Fahrweise erfolgen.

Hinsichtlich der für die Umsetzung (im Labor- oder Produktionsmaßstab) einsetzbaren Reaktionsgefäße gibt es keine besonderen Beschränkungen. Im Falle einer Verwendung eines Reaktors können beispielsweise übliche Rührreaktoren, CSTR (Continuous Stirred Tank Reactor), jeweils mit oder ohne internen und/oder externen Wärmetauscher, ein Rohrreaktor, ein Rohrbündelreaktor oder ein Schlaufenreaktor eingesetzt werden. Ebenso ist es möglich, den Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden. Insbesondere wird die Umsetzung jedoch in einem einzigen Reaktor, insbesondere Rührreaktor, durchgeführt.

Die Verweilzeit der Reaktionsmischung im Reaktor liegt im Allgemeinen im Bereich von 0,1 bis 40 Stunden, bevorzugt im Bereich von 0,1 bis 30 Stunden, weiter bevorzugt im Bereich von 0,1 bis 25 Stunden.

Insbesondere kann die Umsetzung wie folgt durchgeführt werden:

Ein Reaktionsgemisch bestehend aus einem Überschuss an (E/Z)-Cyclohexadec-8-enon und einer im Unterschuss enthaltenen Menge an Cyclopentadec-7/8-enylcarbaldehyd wird in DMF gelöst. Zu der Mischung gibt man als Base Diazabicycloundecen (DBU, 0,5 bis 1,5, z.B. 0.6 eq bezogen auf Carbaldehyd (**XI**)) hinzu. Durch die Mischung wurde kontinuierlich ein Luftstrom geleitet. Der zweizähnige Komplex-Ligand TMEDA (1 bis 5 mol.-%, wie z.B. 2 mol% bezogen auf Carbaldehyd (**XI**)) und Cu(OAc)₂ (1 bis 5 mol.-%, 2 mol%) werden in DMF gelöst. Die Cu-TMEDA-Mischung wird sodann kontinuierlich oder in einer Charge der Reaktionslösung hinzugetropft. Die Reaktion wurde bei 40 bis 60, wie z.B. 50 °C für 10 bis 30 h, wie z.B. 20 h gerührt. Anschließend wird EtOAc und Wasser hinzugegeben. Die wässrige Phase wird mittels 98%iger H₂SO₄ auf pH 4 eingestellt. Die organische Phase wird extrahiert. Anschließend wird die wässrige Phase ggf. nochmals mit EtOAc gewaschen. Die vereinten organischen Phasen werden getrocknet, filtriert und im Vakuum eingeengt.

Der Rückstand wird dann ggf. weiter aufgebarbeite. Beispielsweise führt man eine fraktionierte Destillation durch. Die erfindungsgemäßen Cyclopentadecenone **X** können dadurch von nicht umgesetzten Cyclohexadec-8-enon getrennt werden. Die Cyclopentadecenone **X** können im Anschluss gegebenenfalls von Leichtsiedern, welche durch Destillation nicht abgetrennt wurden, säulenchromatographisch getrennt werden. Hierzu eignet sich z.B. Kieselgel als stationäre Phase und als Laufmittel eine Mischung Cyclohexan: EtOAc, wie z.B. durch Elution mittels eines Stufengradienten 100 : 1 / 30 :1 / 20 : 1. Das Produkt wird bei etwa 80:1 eluiert.

Die Erfindung wird nun unter Bezugnahme auf folgende nichtlimitierende Ausführungsbeispiele näher erläutert:

### Experimenteller Teil

### Methoden:

### Gaschromatographie (GC)

Trennsäule : CP-Wax 52CB 25m x 0,32mm x1,2µm 1ml/min N₂
Bedingungen: 90°-5min-10°/min-240°-30min Inj/Det 200°/250° (Methode A)
Bedingungen: 80°-3°/min-250°- Inj/Det 200°/250° (Methode B) (Nur Beispiel 2)

Probenvolumen: 0,2 ml

### GC/MS

Trennsäule: CP-Wax 52 CB (1.2 µm Filmdicke), Splitverhältnis 10:1
Bedingungen: 80°-3min-240°-30min 0.2µl
MS-Bedingungen: 25-785 amu, 70 eV

### GC/IR

Detektor: MCT/AWellenlänge 650 - 4000 cm⁻¹
Zellen-/Transfertemperatur 250 °C
Scan 6
Resolution 8

### Säulenchromatographie

Verwendet wurde eine Glassäule mit Frittenboden. Die Säule wurde zu 2/3 gepackt mit aufgeschlemmten Kieselgel F₂₅₄. Das Lösungsmittelgemisch wurde mit einem Überdruck von 0.2-0.4 bar durch die Säule gedrückt.

### Beispiel 1: Herstellung eines erfindungsgemäßen Cyclohexadecenon/Cyclopentadecenon-Gemisches

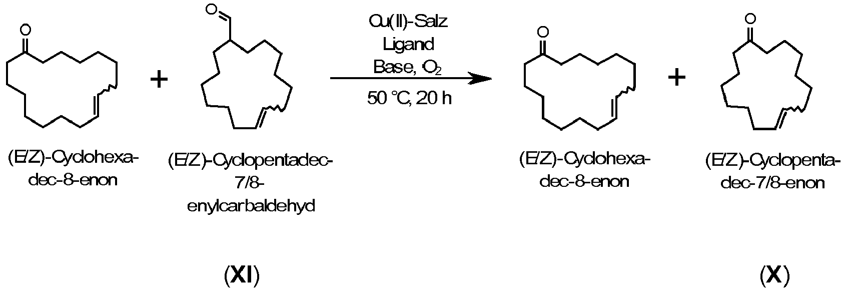

100 g einer Mischung bestehend aus 81% (E/Z)-Cyclohexadec-8-enon und 5.9% Cyclopentadec-7/8-enylcarbaldehyd (z.B. hergestellt nach der PCT/EP2015/072544 der Anmelderin) wurden in 300 ml DMF gelöst. Zu der Mischung wurden 2.3 g Diazabicycloundecen (DBU, 15.2 mmol, 0.6 eq) hinzugegeben. Durch die Mischung wurde kontinuierlich ein Luftstrom geleitet. TMEDA (120 mg, 0.5 mmol, 2 mol%) und Cu(OAc)₂ (90 mg, 0.5 mmol, 2 mol%) wurden in 30 ml DMF gelöst. Die Cu-TMEDA-Mischung wurde über eine Spritzenpumpe kontinuierlich über 6 h oder in einer Charge der Reaktionslösung hinzugetropft. Die Reaktion wurde bei 50 °C für 20 h gerührt. Anschließend wurden 300 ml EtOAc hinzugegeben sowie 200 ml Wasser. Die wässr. Phase wird mittels 98%iger H₂SO₄ auf pH 4 eingestellt. Die organische Phase wurde extrahiert. Anschließend wurde die wässrige Phase noch einmal mit EtOAc (2 x 100 ml) gewaschen. Die vereinten org. Phasen wurden über Na₂SO₄ getrocknet, abfiltriert und im Vakuum eingeengt.

Der Umsatz der Carbaldehyden betrug > 95%. Die Selektivität der Cyclopentadecenone lag bei 95%. Cyclohexadec-8-enon wurde nicht angegriffen.

Der Rückstand wurde mittels fraktionierter Destillation in einer Drehbandkolonne mit einer Stufenzahl von 20 bei 1 mbar Kopfdruck und 125-129 °C Kopf- und 170-180 °C Sumpftemperatur aufgearbeitet. Das Rücklaufverhältnis lag bei 75. Die Cyclopentadec-7/8-enone wurde von Cyclohexadec-8-enon (Ausbeute 85%) getrennt. Die Cyclopentadec-7/8-enone wurden im Anschluss von Leichtsiedern, welche in der Destillation nicht abgetrennt werden konnten, säulenchromatographisch getrennt (Kieselgel, Laufmittel Cyclohexan: EtOAc 100 : 1, 30 :1, 20 : 1), so dass 1 g eines farblosen Öls mit einer Reinheit von 84% (3.7 mmol) erhalten werden konnte.

Anteil:
(E)-Cyclopentadec-8-enon = 54%
(Z)-Cyclopentadec-8-enon = 30%
(E)-Cyclopentadec-7-enon = 0.45%

¹H NMR (500 MHz, CDCl₃, 25 °C): σ = 5.3 (m, 2H), 2.5-2.3 (m, 4H), 2.1-1.9 (m, 4H), 1.7-1.55 (m, 4H), 1.45-1.10 (m, 12H) (des Gemisches)

trans-Cyclopentadec-8-enon I
¹³C-NMR (125 MHz, CDCl₃, 25 °C): σ = 211.98 (C=O), 130.99 (C=C), 41.49 (2xCH₂), 31.80 (2xCH₂), 28.26 (2xCH₂), 28.21 (2xCH₂), 26.84 (2xCH₂), 26.83 (2xCH₂), 22.91 (2xCH₂).

*cis*-Cyclopentadec-8-enon **III**
¹³C-NMR (125 MHz, CDCl₃, 25 °C): σ = 211.93 (C=O), 130.44 (C=C), 41.57 (2xCH₂), 31.80 (2xCH₂), 28.48 (2xCH₂), 27.68 (2xCH₂), 25.74 (2xCH₂), 23.16 (2xCH₂).

MS m/z = 222, 204, 179, 165, 152, 135, 125, 111, 98, 81, 67, 55, 41.

trans-Cyclopentadec-8-enon I
IR (ATR) υ [cm⁻¹] = 3022, 2934, 2864, 1724, 1449, 1356, 1121,969.

*cis*-Cyclopentadec-8-enon **III**
IR (ATR) υ [cm⁻¹] = 3011, 2935, 2866, 1723, 1456, 1354, 716.

Im GC konnte in der Mischung auch *trans*-Cyclopentadec-7-enon **II** mit einem Anteil an 0.45% nachgewiesen werden. Für eine spektroskopische Auswertung der Verbindung war allerdings nicht genügend Material vorhanden. Mittels GC/MS-IR konnte die Verbindung identifiziert werden:

*trans*-Cyclopentadec-7-enon **II**
IR (ATR) u [cm⁻¹] = 3020, 2934, 2864, 1723, 1449, 1353, 969.

Zusätzlich konnte aus einem weiteren Reaktionsansatz mittels GC/MS-IR die Verbindung **IV** identifiziert werden:

*cis*-Cyclopentadec-7-enon **IV**
IR (ATR) u [cm⁻¹] = 3012, 2936, 2866, 1723, 1457, 1353, 714.

### Beispiel 2: Olfaktorische Bewertung eines erfindungsgemäßen Cyclohexadecenon / Cyclopentadecenon-Gemisches

Riechtest: grün, aldehydisch, harsch, gasig, nach Moschus (84% Cyclopentadec-8-enon)

| Riechtest | Befund |
|---|---|
| Riechstreifen <1min | grün, aldehydisch, metallisch, n. Moschus |
| Riechstreifen 10 min | grün, aldehydisch, metallisch, gasig, n. Moschus |
| Riechstreifen 30 min | grün, aldehydisch, metallisch, gasig, n. Moschus |
| Riechstreifen 1 h | grün, aldehydisch, harsch, gasig, n. Moschus |
| Riechstreifen 24 h | grün, aldehydisch, harsch, gasig, n. Moschus |

### Beispiel 3: Herstellung eines erfindungsgemäßen Cyclohexadecenon/Cyclopentadecenon -Gemisches

970 g Cyclopent-7/8-enylcarbaldehyd (**XI**, 12,6 w.-%, 0.52 mol) wurden in 944 g DMF gelöst. Zu der Lösung wurden 47.3 g DBU (0.31 mol, 0.6 eq) hinzugegeben und auf 50 °C erwärmt. Durch die Lösung wurde kontinuierlich ein Luftstrom geleitet. Zu der Lösung wurden 4.7 g Cu(AcO)₂ (25.9 mmol, 5 mol%) und 3.01 g TMEDA (25.9 mmol, 5 mol%) gelöst in 150 ml DMF über eine Zeit von 6 h hinzugetropft. Die Lösung wurde in Summe für 20 h bei 50 °C gerührt. Anschließend wurden 500 ml Wasser und 8 ml 98%ige H₂SO₄ (pH 5) hinzugegeben sowie 500 ml EtOAc. Die Phasen wurden 10 min kräftig gerührt und getrennt. Danach wurde die wäss. Phase erneut mit 500 ml EtOAc extrahiert. Die vereinten org. Phasen wurden mit ges. NaHCO₃-Lösung gewaschen, über Na₂SO₄ getrocknet, abfiltriert und eingeengt. Es wurden 969 g eines dunkelbraunen Rückstandes an (E/Z)-Cyclopentadec-7/8-enon (**X**, 11.7 w.-%) erhalten. Dieser wurde destillativ gereinigt.

### Beispiel 4: Reindestillation Cyclopentadec-7/8-enon:

Zunächst wurde der Reaktionsaustrag aus Beispiel 3 (969 g an (E/Z)-Cyclopentadec-7/8-enon (11.7 w.-%)) mittels Kurzwegverdampfung bei 240 °C im Wärmeträgeröl der Verdampferoberflächen und 5 mbar von Schwersiedern getrennt. Anschließend wurde das Destillat mittels Batchdestillation von den Leichtsiedern getrennt. Die theoretische Stufenzahl der eingesetzten Gewebepackungen Sulzer DX betrug 50. Es wurde bei einem Kopfdruck von 10 mbar mit einem Differenzdruck über die Kolonne von 8 mbar gearbeitet. Zur Abtrennung der (E/Z)-Cyclopentadec-7/8-enon betrug das Rücklaufverhältnis 50. Die Cyclopentadec-7/8-enon wurden fraktioniert destilliert. Nach deren Abtrennung wurde das Rücklaufverhältnis zur Destillation des Cyclohexadec-8-enons auf 20 reduziert. Die Sumpftemperatur stieg während der Destillation von 204 auf 220 °C an. Die Kopftemperatur stieg von 160 auf 175 °C an.

Mittels der fraktionierten Destillation konnte die Reinheit an (E/Z)-Cyclopentadec-7/8-enon von 11.5 w.-% auf 94-98% (FI.-%) erhöht werden. Das Verhältnis von *trans-* zu *cis-* Cyclopentadec-8-enon lag hier bei 4:1 (Fr. 3) bzw. 2:1 (Fr. 4).

### Beispiel 5: Olfaktorische Einstufung des 98%igen Cyclopentadec-7/8-enon-Musters

| | |
|---|---|
| Proben "Fr 3" und "Fr.4" aus Beispiel 4 | |
| Intensität | 4 |
| Moschus | 6 |
| Süß, pudrig | 4 |
| Trockenobst | 2 |

Auf die Offenbarung der hierin erwähnten Druckschriften wird ausdrücklich Bezug genommen.

## Patentansprüche

1. Verfahren zur Herstellung einer makrocyclischen Ketoverbindung der allgemeinen Formel **X** 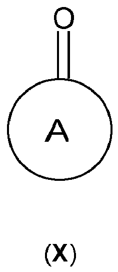 worin A für einen cycloaliphatischen Kohlenwasserstoffrest mit m Ringkohlenstoffatomen steht, wobei m für einen ganzzahligen Wert von 13 bis 17 steht, und gegebenenfalls n C=C-Doppelbindungen aufweist, wobei n für einen ganzzahligen Wert von 1, 2 oder 3 steht, wobei man
a) eine cycloaliphatische Carbaldehydverbindung der Formel **XI** 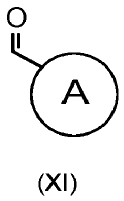 worin A die oben angegebenen Bedeutungen besitzt, oxidativ decarbonyliert; wobei man die Verbindung der Formel **XI** in Gegenwart eines Cu-(II)-Katalysators und molekularem Sauerstoff umsetzt; und gegebenenfalls
b) wenigstens eine Verbindung der Formel **X** aus dem Reaktionsgemisch isoliert.

2. Verfahren nach Anspruch 1, wobei man den Katalysator in situ bildet, indem man zu einem Cu-(II)-Salz einen Liganden gibt.

3. Verfahren nach einem der vorhergehenden Ansprüche, wobei in den Verbindungen der Formel **X** und **XI** m für 15 und/oder n für 1 steht.

4. Verfahren nach einem der vorhergehenden Ansprüche, wobei man (E/Z)-Cyclopentadec-8-enyl-1-carbaldehyd oder eine Mischung aus (E/Z)-Cyclopentadec-8-enyl- und (E/Z)-Cyclopentadec-7-enyl-1-carbaldehyd oder eine diese Verbindung enthaltendes Stoffgemisch einsetzt.

5. Verfahren nach Anspruch 4, wobei man wenigstens eine Verbindung, ausgewählt unter (E/Z)-Cyclopentadec-8-en-1-on und (E/Z)-Cyclopentadec-7-en-1-on erhält.

6. Verfahren nach Anspruch 5, wobei man wenigstens eine Verbindung ausgewählt unter Verbindungen der folgenden Formeln I, **II, III** und **IV** 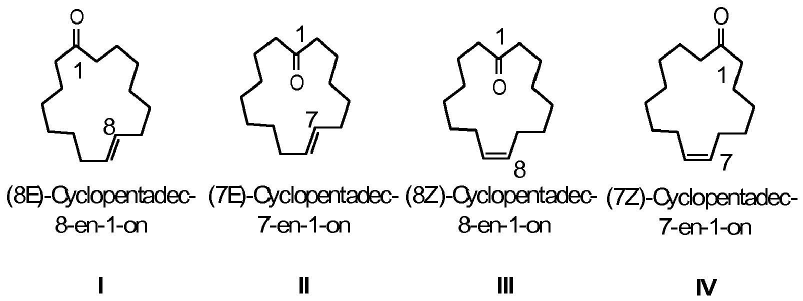 erhält.

7. Verfahren nach einem der vorhergehenden Ansprüche, wobei man in Stufe a) ein Reaktionsprodukt einsetzt, das man erhält aus der Distickstoffmonoxid-Oxidation einer cycloaliphatische Verbindung der Formel **XII** 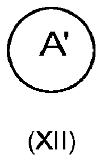 worin A' für einen cycloaliphatischen Kohlenwasserstoffrest mit m+1 Ringkohlenstoffatomen steht, wobei m für einen ganzzahligen Wert von 13 bis 17 steht, und gegebenenfalls n+1 C=C-Doppelbindungen aufweist, und worin n für einen ganzzahligen Wert von 1, 2 oder 3 steht.

8. Verfahren nach Anspruch 7, worin in der Verbindung der Formel **XII** m für 15 und n für 1 steht.

9. Verfahren nach Anspruch 8, worin die Verbindung der Formel **XII** Cyclohexadeca-1,9-dien ist.

10. Verfahren zur Herstellung von Globanon ((E/Z)-Cyclohexadec-8-en-1on), wobei man
a) Cyclohexadeca-1,9-dien mit Distickstoffmonoxid oxidiert, wobei man ein Reaktionsgemisch erhält, welches Globanon im Gemisch wenigstens eine Verbindung ausgewählt unter (E/Z)-Cyclopentadec-8-enyl-1-carbaldehyd und (E/Z)-Cyclopentadec-7-enyl-1-carbaldehyd enthält; wobei man nichtumgesetztes Cyclohexadeca-1,9-dien gegebenenfalls abtrennt;
b) das Reaktionsgemisch aus Stufe a) einer oxidativen Decarbonylierungsreaktion nach einem der Ansprüche 1 bis 2 unterzieht; und
c) Globanon von den dabei gebildeten Cyclopentadecenonen I - **IV** trennt.

11. Stoff oder Stoffgemisch enthaltend wenigstens eine makrocyclische Ketoverbindung der obigen allgemeinen Formel **X.**

12. Stoffgemisch nach Anspruch 11, umfassend wenigstens zwei Verbindungen, ausgewählt unter (E/Z)-Cyclopentadec-8-en-1-on und (E/Z)-Cyclopentadec-7-en-1-on.

13. Stoffgemisch nach Anspruch 12, umfassend wenigstens zwei Verbindungen ausgewählt unter Verbindungen der folgenden Formeln I, **II** und **III** 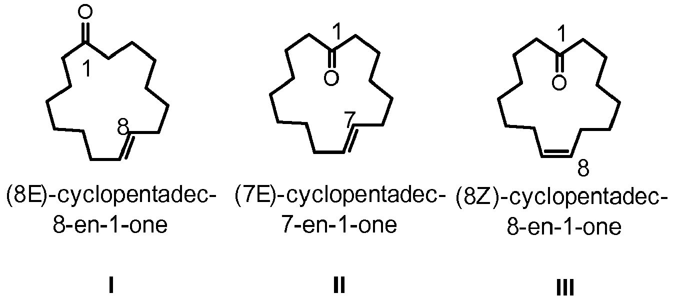 oder umfassend wenigstens zwei Verbindungen ausgewählt unter Verbindungen der folgenden Formeln I, **II, III** und **IV**

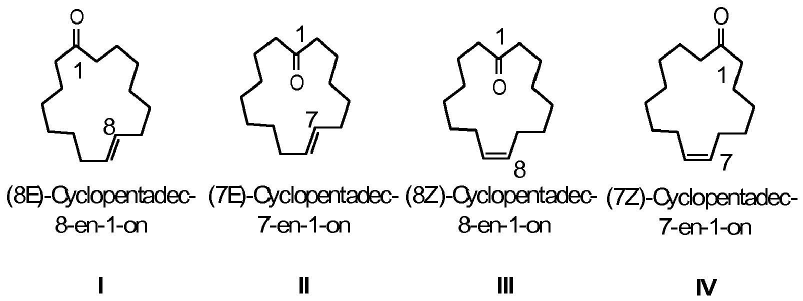

14. Verwendung wenigstens eines Stoffes oder Stoffgemisches nach einem der Ansprüche 11 bis 13 als Aromachemikalie, insbesondere als Riechstoff.

15. Verwendung nach Anspruch 18 eines Gemischs, im Wesentlichen enthaltend (E)-Cyclopentadec-8-en-1-on (I) und (Z)-Cyclopentadec-8-en-1-on (**III**).

16. Verwendung nach Anspruch 15, wobei das Molverhältnis von (I) zu (**III**) im Bereich von etwa 1:1 bis etwa 5:1 liegt

17. Verwendung nach einem der Ansprüche 14 bis 16 zur Erzeugung einer Moschusnote in einer Riechstoffzusammensetzung.

18. Riechstoffzusammensetzung, enthaltend wenigstens einen Stoff oder ein Stoffgemisch gemäß der Definition in einem der Ansprüche 11 bis 13 oder hergestellt nach einem der Verfahren gemäß den Ansprüchen 1 bis 10.

19. Mittel enthaltend wenigstens einen Stoff oder ein Stoffgemisch gemäß der Definition in einem der Ansprüche 11 bis 13 oder hergestellt nach einem der Verfahren gemäß den Ansprüchen 1 bis 10.
